(19) Europäisches Patentamt / European Patent Office / Office européen des brevets

(11) **EP 3 769 750 A1**

(12) **EUROPEAN PATENT APPLICATION**

(43) Date of publication:
27.01.2021 Bulletin 2021/04

(21) Application number: 20184629.2

(22) Date of filing: 08.07.2020

(51) Int Cl.:
*A61K 8/73* (2006.01)  *A61Q 19/00* (2006.01)
*A61Q 19/08* (2006.01)  *A61K 31/728* (2006.01)
*A61K 47/36* (2006.01)  *A61P 17/00* (2006.01)
*A61K 8/04* (2006.01)  *C08L 5/08* (2006.01)
*C08B 37/08* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 24.07.2019  KR 20190089762

(71) Applicant: **Amorepacific Corporation**
**Seoul 04386, (KR)**

(72) Inventors:
• CHO, Sungyeon
  **17074 Gyeonggi-do (KR)**
• JANG, Jiwook
  **17074 Gyeonggi-do (KR)**
• JEONG, Haewon
  **17074 Gyeonggi-do (KR)**
• CHOI, Kyungho
  **17074 Gyeonggi-do (KR)**

(74) Representative: **Agasse, Stéphane et al**
**Cabinet GERMAIN & MAUREAU**
**B.P. 6153**
**69466 Lyon Cedex 06 (FR)**

(54) **A HYALURONIC ACID COMPOSITION FOR EXTERNAL USE ON SKIN AND ITS USE**

(57) The present disclosure provides a hyaluronic acid composition for external use on skin, which includes crosslinked hyaluronic acid having a water swelling degree of 50-100 times in an amount of 0.5 wt% or more on dry weight basis, based on the total weight of the composition. While the hyaluronic acid composition for external use on skin includes crosslinked hyaluronic acid at a high concentration, it shows excellent spreadability, and shows an excellent skin moisturizing effect and low stickiness.

EP 3 769 750 A1

**Description**

[Technical Field]

The present disclosure relates to a hyaluronic composition for external use on skin.

[Background Art]

Polysaccharide is a sugar complex and includes monosaccharides bound through glycoside bonding. Particularly, hyaluronic acid, a kind of polysaccharide, has been used widely in the field of cosmetics and medicines.
Such hyaluronic acid is a substance forming the body and found in the body fluid, bovine eyeball, cockscomb, cushioning tissues of animals, placenta, or the like, and is present at a high concentration particularly in the connective tissues and skin.
In addition, hyaluronic acid is capable of containing water in an amount corresponding to approximately 1000 times of its weight. Thus, it has been used widely as a moisturizing agent for cosmetics to prevent the skin from drying. In the field of skin cosmetics, hyaluronic acid has been used as a dermal filler inserted to a specific site to extend soft tissues, thereby improving wrinkles or correcting face lines. Further, hyaluronic acid has excellent viscoelasticity, biocompatibility and biodegradability, and thus has been used widely as an agent for treating osteoarthritis, agent for treating wounds, adjuvant for eyeball surgery, agent for preventing post-surgical adhesion between tissues, or the like, in addition to the skin cosmetic field.
However, according to the related art, it has been reported that hyaluronic acid is significantly slippery and shows high stickiness, and thus is hardly used at a high concentration, particularly in the case of preparation of cosmetic products.

**[Disclosure]**

[Technical Problem]

A technical problem to be solved by the present disclosure is to provide a hyaluronic acid composition for external use on skin which shows excellent spreadability to provide an excellent skin moisturizing effect, and has low stickiness.

[Technical Solution]

In one general aspect, there is provided a hyaluronic acid composition for external use on skin which includes crosslinked hyaluronic acid in an amount of 0.5 wt% or more on dry weight basis, based on the total weight of the composition, wherein the crosslinked hyaluronic acid has a water swelling degree of 50-100 times, wherein the water swelling degree is a ratio of the weight of gel determined after storing the crosslinked hyaluronic acid in water for 12 hours based on its dry weight.

[Advantageous Effects]

According to the embodiments of the present disclosure, there is provided a hyaluronic acid composition for external use on skin which includes a high concentration of crosslinked hyaluronic acid to provide improved spreadability and an excellent skin moisturizing effect, and shows low stickiness and a fresh feeling of use.

[Brief Description of Drawings]

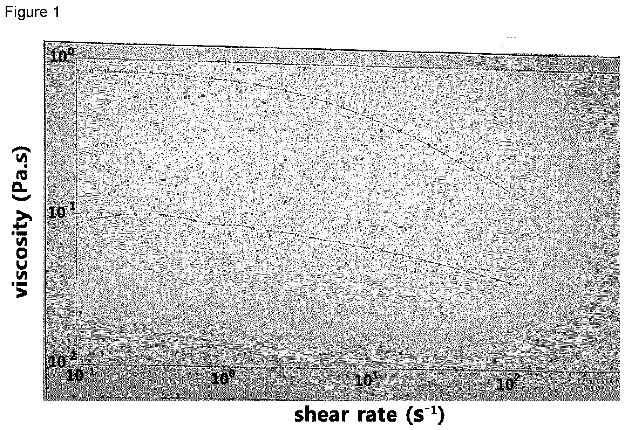

FIG. 1 is a graph illustrating the results of determination of viscosity of the hyaluronic composition for external use on skin according to an embodiment of the present disclosure as compared to the conventional hyaluronic acid-containing composition.

[Best Mode]

Throughout the specification, the expression 'a part 「comprises」 an element' does not preclude the presence of any additional elements but means that the part may further comprise the other elements, unless otherwise stated.
As used herein, 'composition for external use on skin' is a generic term covering any compositions applied from the outside of the skin, and includes various formulations of cosmetic products and medicines.

Hereinafter, the present disclosure will be explained in detail.

In one aspect of the present disclosure, there is provided a hyaluronic acid composition for external use on skin which includes crosslinked hyaluronic acid in an amount of 0.5 wt% or more on dry weight basis, based on the total weight of the composition, wherein the crosslinked hyaluronic acid has a water swelling degree of 50-100 times, wherein the water swelling degree is a ratio of the weight of gel determined after storing the crosslinked hyaluronic acid in water for 12 hours based on its dry weight.

According to the related art, hyaluronic acid is an ingredient effective for moisturization but has a disadvantage in that it is severely slippery and sticky and thus is hardly used at a high concentration in a cosmetic formulation.

To overcome the disadvantage, the inventors of the present disclosure have found a hyaluronic acid composition for external use on skin which has an optimized water swelling degree of the crosslinked hyaluronic acid so that the crosslinked hyaluronic acid may be included at a high concentration of 0.5 wt% or more on dry weight basis, shows improved spreadability and an excellent skin moisturizing effect, and has low stickiness to provide a fresh feeling of use.

The crosslinked hyaluronic acid may be obtained by crosslinking hyaluronic acid in a basic solution by using an epoxide-based crosslinking agent, dipping the crosslinked product in water so that a water swelling degree of 50-100 times may be obtained, and dispersing the resultant product in a solvent.

Particularly, the crosslinking may be carried out at 20-50°C for 10-30 hours. For example, the crosslinking may be carried out at 25°C or higher, 30°C or higher, or 35°C or higher, and 50°C or lower, 47°C or lower, 44°C or lower, or 40°C or lower. In addition, the crosslinking may be carried out for 15 hours or more, 20 hours or more, or 25 hours or more, and 28 hours or less, 26 hours or less, or 24 hours or less.

Herein, since the crosslinked hyaluronic acid may cause irritation to the human body due to the ether bonding, the crosslinked hyaluronic acid may be washed.

According to an embodiment, the term 'crosslinking' may refer to ether bonding between the OH group of hyaluronic acid and the epoxide-based crosslinking agent.

According to an embodiment, the crosslinked hyaluronic acid may be crosslinked with an epoxide-based crosslinking agent.

According to an embodiment, the crosslinked hyaluronic acid may be crosslinked with at least one selected from the group consisting of 1,4-butanediol diglycidyl ether (BDDE), ethylene glycol diglycidyl ether (EGDGE), 1-(2,3-epoxypropyl)-2,3-epoxycyclohexane, polyethylene diglycidyl ether (PEGDE) and 1,2-ethanediol diglycidyl ether. Preferably, the epoxide-based crosslinking agent may be 1,4-butanediol diglycidyl ether (BDDE).

According to an embodiment, the hyaluronic acid composition for external use on skin may be provided in the form of gel.

According to an embodiment, the hyaluronic acid composition for external use on skin may have a $\tan\delta$ value of 0.5-1, as determined according to the following Formula 1:

$$[\text{Formula 1}]$$

$$\text{Tan}\delta = G''/G'$$

wherein G" is viscosity of the hyaluronic acid composition for external use on skin, and G' is elasticity of the hyaluronic acid composition for external use on skin.

Herein, each of G" and G' is expressed in the unit of Pa, and may be determined by using a rheometer with a 20 mm plate at 25°C under the conditions of a frequency range of 0.1-10 Hz, shear stress of 1 Pa and a gap of 1 mm, and reading G' and G" values at 1Hz.

For example, the $\tan\delta$ value may be 0.6 or more, 0.7 or more, or 0.8 or more, and 0.95 or less, or 0.9 or less. When the $\tan\delta$ value is less than 0.5, the composition is nearly a hard solid and shows poor spreadability. When the $\tan\delta$ value is larger than 1, it is nearly liquid, not gel, and thus is not suitable for formation of a moisturizing film.

The water swelling degree is a ratio of the weight of gel determined after storing the crosslinked hyaluronic acid in water for 12 hours based on its dry weight. Particularly, the water swelling degree may be calculated according to the following Formula 2 as a ratio of the weight of crosslinked hyaluronic acid (crosslinked HA gel) swelled completely after storing it in water for 12 hours based on the weight of crosslinked hyaluronic acid (crosslinked HA gel) after drying.

$$[\text{Formula 2}]$$

$$\text{Swelling degree (times)} = \text{weight of swelled crosslinked HA gel} \,/\, \text{weight of dried crosslinked HA gel}$$

For example, the crosslinked hyaluronic acid may have a water swelling degree of 55 times or more, 60 times or more, 65 times or more, or 70 time or more, and 95 times or less, 90 times or less, or 85 times or less.

For example, the hyaluronic acid composition for external use on skin may include crosslinked hyaluronic acid in an amount of 0.6 wt% or more, 0.7 wt% or more, 0.8 wt% or more, or 0.9 wt% or more, on dry weight basis, based on the total weight of the hyaluronic acid composition.

According to an embodiment, the crosslinked hyaluronic acid may have a particle size of 100 $\mu$m or more. For example, the crosslinked hyaluronic acid may have a particle size of 100 $\mu$m or more, 150 $\mu$m or more, or 200 $\mu$m or more, and 500 $\mu$m or less, 400 $\mu$m or less, or 300 $\mu$m or less. Since the particle size is 100 $\mu$m or more, it is possible to maintain a watery feeling for a longer time and to provide a unique feeling of use to the user upon skin application. In addition, when the particle size is larger than 500 $\mu$m, dirt scrubbing may occur.

According to an embodiment, the hyaluronic acid composition for external use on skin may further include a solvent in an amount of 20-70 wt% based on the total weight of the composition.

For example, the solvent may be a water soluble solvent, such as distilled water or a lower alcohol, such as ethanol.

For example, the solvent may be used in an amount of 22 wt% or more, 24 wt% or more, 26 wt% or more, 28 wt% or more, or 30 wt% or more, and 68 wt% or less, 66 wt% or less, 64 wt% or less, 62 wt% or less, or 60 wt% or less. When the solvent is used in an amount less than 20 wt%, spreadability is degraded so that a stiff feeling of use may be provided upon skin application. When the solvent is used in an amount larger than 60 wt%, the hyaluronic acid composition for external use on skin cannot retain its gel-like shape but roll down.

According to an embodiment, the hyaluronic acid composition may be for skin moisturization.

According to an embodiment, the composition for external use on skin may be a cosmetic composition, and the outer shape of the cosmetic composition includes a cosmetically or dermatologically acceptable medium or base. The composition may include any formulations suitable for local application. For example, the composition may be provided in the form of a solution, gel, solid, dry slurry product, emulsion prepared by dispersing an oil phase in an aqueous phase, suspension, microemulsion, microcapsules, microgranules or ionic (liposome) and non-ionic sachet dispersant, or provided as cream, skin, lotion, powder, ointment, spray or conceal stick. Such formulations may be obtained by the method generally known to those skilled in the art. In addition, the cosmetic composition may be used in the form of foam or an aerosol composition further including a compressed propellant.

The cosmetic composition according to an embodiment of the present disclosure is not limited to any particular formulation. For example, the cosmetic composition may be formulated into cosmetic products, such as ampoule, cream, skin softener, skin astringent, skin nutrient, nutrient cream, massage cream, essence, eye cream, eye essence, cleansing cream, cleansing foam, cleansing water, cleansing tissue containing the cosmetic composition, pack, powder, body lotion, body cream, body oil and body essence.

When the formulation is paste, cream or gel, carrier ingredients that may be used include animal fibers, vegetable fibers, wax, paraffin, starch, tragacanth, cellulose derivatives, polyethylene glycol, silicone, bentonite, silica, talc or zinc oxide.

When the formulation is powder or spray, carrier ingredients that may be used include lactose, talc, silica, aluminum hydroxide, calcium silicate or polyamide powder. Particularly, in the case of spray, it may further include a propellent, such as chlorofluorohydrocarbon, propane/butane or dimethyl ether.

When the formulation is a solution or emulsion, carrier ingredients that may be used include a solvent, solvating agent or emulsifying agent, such as water, ethanol, isopropanol, ethyl carbonate, ethyl acetate, benzyl alcohol, benzyl benzoate, propylene glycol, 1,3-butylglycol oil, glycerol fatty acid ester, polyethylene glycol or sorbitan fatty acid ester.

When the formulation is a suspension, carrier ingredients that may be used include a liquid diluent, such as water, ethanol or propylene glycol, a suspending agent, such as ethoxylated isostearyl alcohol, polyoxyethylene sorbitol ester or polyoxyethylene sorbitan ester, microcrystalline cellulose, aluminum metahydroxide, bentonite, agar, tragacanth, or the like.

When the formulation is a surfactant-containing cleanser, carrier ingredients that may be used include aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, aliphatic alcohol, fatty acid glyceride, fatty acid diethanolamine, vegetable oil, lanoline derivative, ethoxylated glycerol fatty acid ester, or the like.

The cosmetic composition according to an embodiment of the present disclosure may further include functional additives and include other ingredients used currently in a cosmetic composition. The functional additive may include an ingredient selected from the group consisting of water soluble vitamins, oil soluble vitamins, polymer peptides, polymer polysaccharides, spingolipids and seaweed extract.

In addition to the above-mentioned ingredients, the cosmetic composition according to an embodiment of the present disclosure may further include an oil and fat ingredient, moisturizing agent, emollient, surfactant, organic and inorganic pigments, organic powder, preservative, sterilizing agent, antioxidant, plant extract, pH modifier, alcohol, colorant, fragrance, blood flow-stimulating agent, coolant, anti-perspirant, purified water, or the like.

According to an embodiment, the hyaluronic acid composition for external use on skin may be a pharmaceutical composition. When applying the hyaluronic acid composition for external use on skin to medicines, an inorganic or organic carrier used currently for the active ingredient used in the present disclosure may be added so that the composition may be formulated into a semisolid or liquid formulation for parenteral administration. The pharmaceutical composition according to the present disclosure may be administered through a parenteral, rectal, local, transdermal, intravenous, intramuscular, intraperitoneal, subcutaneous route, or the like.

Formulations for parenteral administration may include injections, fillers, drops, ointment, lotion, spray, suspension, emulsion, suppositories, or the like. The composition according to the present disclosure may be easily formulated with an active ingredient according to the method generally known to those skilled in the art, wherein a surfactant, vehicle, colorant, fragrance, preservative, stabilizer, buffer, suspending agent, or other conventional adjuvants may be used suitably.

The effective administration dose of the pharmaceutical composition according to an embodiment of the present disclosure may be varied depending on age, sex, body weight, pathological condition and severity of a subject to be administered, administration route or judgement of a prescriber. Determination of a suitable dose may be made by those skilled in the art based on the above-mentioned factors. For example, the pharmaceutical composition may be administered in a daily dose of 0.1-100 mg/kg/day, particularly 5-50 mg/kg/day, but is not limited thereto.

In another aspect of the present disclosure, there is provided a method for improving skin moisturizing including applying a hyaluronic acid composition for external use on skin onto the skin, wherein the hyaluronic acid composition for external use on skin includes crosslinked hyaluronic acid in an amount of 0.5 wt% or more on dry weight basis, based on the total weight of the composition, wherein the crosslinked hyaluronic acid has a water swelling degree of 50-100 times, wherein the water swelling degree is a ratio of the weight of gel determined after storing the crosslinked hyaluronic acid in water for 12 hours based on its dry weight.

In still another aspect of the present disclosure, there is provided use of a hyaluronic acid composition for manufacturing a skin moisturizing composition for external use on skin, wherein the hyaluronic acid composition includes crosslinked hyaluronic acid in an amount of 0.5 wt% or more on dry weight basis, based on the total weight of the composition, wherein the crosslinked hyaluronic acid has a water swelling degree of 50-100 times, wherein the water swelling degree is a ratio of the weight of gel determined after storing the crosslinked hyaluronic acid in water for 12 hours based on its dry weight.

In yet another aspect of the present disclosure, there is provided a hyaluronic acid composition for skin moisturization, wherein the hyaluronic acid composition includes crosslinked hyaluronic acid in an amount of 0.5 wt% or more on dry weight basis, based on the total weight of the composition, wherein the crosslinked hyaluronic acid has a water swelling degree of 50-100 times, wherein the water swelling degree is a ratio of the weight of gel determined after storing the crosslinked hyaluronic acid in water for 12 hours based on its dry weight.

Hereinafter, the present disclosure will be explained in more detail with reference to examples. However, the following examples are for illustrative purposes only. In addition, it will be apparent to those skilled in the art that the scope of the present disclosure is not limited to the following examples.

**Example** 1 - **Preparation of Hyaluronic Acid Composition for External Use on Skin and Determination of Skin Water Content/Spreadability Thereof**

The crosslinked hyaluronic acid compositions for external use on skin of Examples 1-3 and Comparative Examples 1 and 2 were prepared by using crosslinked hyaluronic acid having a different water swelling degree according to the following Tables 1 and 2.

Particularly, sodium hyaluronate was dissolved in a solution containing 1,4-butanediol diglycidyl ether (BDDE, Sigma Aldrich) and 0.24 N NaOH and crosslinking was carried out at 37°C for 23 hours. The crosslinked product was washed with distilled water sufficiently, and pulverized into a size of 250 $\mu$m.

Water swelling degree was calculated according to the following Formula 2 and controlled by the crosslinking reaction.

[Formula 2]

Water swelling degree (times) = Weight of swelled crosslinked HA gel / Weight of dried crosslinked HA gel

Then, each pulverized crosslinked hyaluronic acid was used in an amount of 0.5 wt% as determined by a ratio of its dry weight based on the total weight of each composition to provide each of the crosslinked hyaluronic acid compositions for external use on skin according to Examples 1-3 and Comparative Examples 1 and 2.

In addition, the skin water content as shown in Table 2 was determined by cleansing both upper arms of each of 30-40 aged male subjects with cleansing foam, removing water from the upper arms, applying 0.2 mL of each of the crosslinked hyaluronic acid compositions for external use on skin according to Examples 1-3 and Comparative Examples 1 and 2 to the upper arms, rubbing the upper arms well, and measuring the skin water content by using a corneometer available from C+K electronic Co., after 1 hour.

Further, the spreadability in Table 2 was determined by checking how easy each composition is applied, when rubbing the upper arms after applying 0.2 mL of each of the crosslinked hyaluronic acid compositions for external use on skin according to Examples 1-3 and Comparative Examples 1 and 2 to the upper arms.

[Table 1]

| No. | Ingredients | Ex. 1 | Ex. 2 | Ex. 3 | Comp. Ex. 1 | Comp. Ex. 2 |
|---|---|---|---|---|---|---|
| 1 | Solvent: D.I. water | 47.2 | 55.5 | 30.5 | 65.3 | 0 |
| 2 | Ion controlling agent: E.D.T.A-2NA | 0.02 | 0.02 | 0.02 | 0.02 | 0.02 |
| 3 | Wetting agent: AMINOCOAT | 1.0 | 1.0 | 1.0 | 1.0 | 1.0 |
| 4 | Lipid portion: TREHALOSE | 1.0 | 1.0 | 1.0 | 1.0 | 1.0 |
| 5 | Moisturizer: GLYCERINE | 5.0 | 5.0 | 5.0 | 5.0 | 5.0 |
| 6 | Moisturizer: KONLUB DR-701C (Polyoxyalkylene Glycol Mono Alkyl Ester) | 1.0 | 1.0 | 1.0 | 1.0 | 1.0 |
| 7 | Moisturizer: BUTYLENE GLYCOL (1,3) | 7.0 | 7.0 | 7.0 | 7.0 | 7.0 |
| 8 | Crosslinked hyaluronic acid gel (HA gel) | 33.3 | 25.0 | 50.0 | 15.2 | 80.5 |
| 9 | Solvent: Ethanol | 3.0 | 3.0 | 3.0 | 3.0 | 3.0 |
| 10 | Oil: EMALEX HC-60(PEG-60 Hydrogenated Castor Oil) | 0.5 | 0.5 | 0.5 | 0.5 | 0.5 |
| 11 | Emulsifier: CEODOL 2016(Octyldodeceth-16) | 0.3 | 0.3 | 0.3 | 0.3 | 0.3 |
| 12 | Preservative: Hydrolite 6 O (1,2-Hexanediol) | 0.7 | 0.7 | 0.7 | 0.7 | 0.7 |

[Table 2]

| | Ex. 1 | Ex. 2 | Ex. 3 | Comp. Ex. 1 | Comp. Ex. 2 |
|---|---|---|---|---|---|
| Water swelling degree (times) of crosslinked hyaluronic acid gel | 70 | 50 | 100 | 30 | 150 |
| Skin water content | 88.0 | 72.3 | 78.7 | 44.3 | 48.1 |
| Spreadability | Good | Good | Good | Roll down | Stiff |

As can be seen from the above results, Examples 1-3 have a significantly high skin water content, while Comparative Examples 1 and 2 having a skin water content of 30 times and 150 times, respectively, show a skin water content of about 40. This suggests that skin water content depends on water swelling degree.

In addition, after checking spreadability, it can be seen that an excessively low water swelling degree causes the composition to roll down upon skin application, while an excessively high water swelling degree results in a relative decrease in content of solvent contained in the composition to cause stiff spreading.

## Example 2 - Determination of Viscosity Depending on Crosslinking of Hyaluronic Acid

To compare the viscosity of a commercially available hyaluronic acid ampoule (IOPE hyaluronic ampoule, containing non-crosslinked hyaluronic acid) with that of the hyaluronic acid composition (Example 1) for external use on skin according to an embodiment of the present disclosure, viscosity was measured by using a rheometer at 25°C with a 40 mm 1 cone in a range of 0.1-20 s$^{-1}$. The results are shown in FIG. 1.

Referring to FIG. 1, the composition according to Example 1 (lower graph in FIG. 1) has lower viscosity as compared to the commercially available hyaluronic acid ampoule (upper graph in FIG. 1). Thus, it can be seen that the composition according to an embodiment of the present disclosure is less sticky, even though it includes the same content of crosslinked hyaluronic acid.

**Example 3 - Determination of Tanδ depending on Particle Size**

To compare the tanδ value depending on particle size of a commercially available hyaluronic acid ampoule (IOPE hyaluronic ampoule, containing non-crosslinked hyaluronic acid) with that of the hyaluronic acid composition (Example 1) for external use on skin according to an embodiment of the present disclosure, tanδ was determined by using a rheometer at 25°C with a 20 mm plate in a frequency range of 0.1-10 Hz under the conditions of a shear stress of 1 Pa and a gap of 1 mm. Herein, G' and G" values were measured at 1 Hz and tanδ was calculated according to Formula 1. The results are shown in the following Table 3.

$$[Formula\ 1]$$

$$Tanδ = G"/G'$$

wherein G" is viscosity of the hyaluronic acid composition for external use on skin, and G' is elasticity of the hyaluronic acid composition for external use on skin.

[Table 3]

|  | Particle size | G' (Elasticity) | G" (Viscosity) | Tanδ (G"/G') |
|---|---|---|---|---|
| Crosslinked hyaluronic acid ampoule (Ex. 1) | 250 $\mu$m | 34.85 Pa | 28.63 Pa | 0.82 |
| Commercially available hyaluronic acid ampoule | 0 $\mu$m | 2.146 Pa | 3.584 Pa | 1.67 |

Referring to the above results, as compared to the commercially available hyaluronic acid ampoule (containing non-crosslinked hyaluronic acid, no particle is present) having a particle size of 0 $\mu$m, the crosslinked hyaluronic acid composition for external use on skin having a particle size of 250 $\mu$m according to the present disclosure has a tanδ value smaller than 1 to provide a particle-like feeling. In addition, the tanδ value of the composition according to the present disclosure is larger than 0.5 to provide easy spreadability, since the composition is not hard solid.
On the contrary, since the commercially available hyaluronic acid ampoule has a tanδ value larger than 1, it is an ampoule having liquid-like property. Thus, it is likely that the ampoule liquid rolls down upon skin application.

**Example 4** - **Determination of Water Retention Depending on Crosslinking of Hyaluronic Acid**

To determine the water retention of the composition according to the present disclosure as compared to a low-viscosity ampoule (ampoule containing no hyaluronic acid) and commercially available ampoule (IOPE hyaluronic ampoule, containing non-crosslinked hyaluronic acid), the composition according to Example 1 was applied to the skin of each of 30-40 aged male subjects, and water retention was determined by using a corneometer available from C+K electronic Co., after 16 hours. The results are shown in the following Table 4.

[Table 4]

|  | Water retention |
|---|---|
| Low-viscosity ampoule | 15 |
| Commercially available ampoule | 17.5 |
| Crosslinked hyaluronic acid ampoule (Ex. 1) | 30 |

As can be seen from the foregoing, the crosslinked hyaluronic acid ampoule according to the present disclosure has a water retention of 30%, which is significantly higher as compared to the low-viscosity ampoule containing no hyaluronic acid or commercially available hyaluronic acid ampoule containing non-crosslinked hyaluronic acid.

Embodiment 1: A hyaluronic acid composition for external use on skin including crosslinked hyaluronic acid in an amount of 0.5 wt% or more on dry weight basis, based on the total weight of the hyaluronic acid composition, wherein the crosslinked hyaluronic acid has a water swelling degree of 50-100 times, wherein the water swelling degree is a ratio of the weight of gel determined after storing the crosslinked hyaluronic acid in water for 12 hours based on its dry weight.

Embodiment 2: The hyaluronic acid composition for external use on skin as defined in Embodiment 1, wherein the crosslinked hyaluronic acid is crosslinked with an epoxide-based crosslinking agent.

Embodiment 3: The hyaluronic acid composition for external use on skin as defined in Embodiment 1 or 2, wherein the crosslinked hyaluronic acid is crosslinked with at least one selected from the group consisting of 1,4-butanediol diglycidyl ether (BDDE), ethylene glycol diglycidyl ether (EGDGE), 1-(2,3-epoxypropyl)-2,3-epoxycyclohexane, polyethylene diglycidyl ether (PEGDE) and 1,2-ethanediol diglycidyl ether.

Embodiment 4: The hyaluronic acid composition for external use on skin as defined in any one of Embodiments 1 to 3, wherein the hyaluronic acid composition is provided in the form of gel.

Embodiment 5: The hyaluronic acid composition for external use on skin as defined in any one of Embodiments 1 to 4, wherein the hyaluronic acid composition has a tanδ value of 0.5-1, as determined according to the following Formula 1:

$$[Formula\ 1]$$

$$Tan\delta = G''/G'$$

wherein G" is viscosity of the hyaluronic acid composition, and G' is elasticity of the hyaluronic acid composition.

Embodiment 6: The hyaluronic acid composition for external use on skin as defined in any one of Embodiments 1 to 5, wherein a particle size of the crosslinked hyaluronic acid is 100 μm or more.

Embodiment 7: The hyaluronic acid composition for external use on skin as defined in any one of Embodiments 1 to 6, wherein the hyaluronic acid composition further includes a solvent in an amount of 20-70 wt% based on the total weight of the composition.

Embodiment 8: The hyaluronic acid composition for external use on skin as defined in any one of Embodiments 1 to 7, wherein the hyaluronic acid composition is a cosmetic composition.

Embodiment 9: A non-therapeutic use of a hyaluronic acid composition for skin moisturization,
wherein the hyaluronic acid composition comprises crosslinked hyaluronic acid in an amount of 0.5 wt% or more on dry weight basis, based on the total weight of the hyaluronic acid composition,
wherein the crosslinked hyaluronic acid has a water swelling degree of 50-100 times, wherein the water swelling degree is a ratio of the weight of gel determined after storing the crosslinked hyaluronic acid in water for 12 hours based on its dry weight.

Embodiment 10: The non-therapeutic use according to Embodiment 9, wherein the crosslinked hyaluronic acid is crosslinked with an epoxide-based crosslinking agent,
preferably,
the crosslinked hyaluronic acid is crosslinked with at least one selected from the group consisting of 1,4-butanediol diglycidyl ether (BDDE), ethylene glycol diglycidyl ether (EGDGE), 1-(2,3-epoxypropyl)-2,3-epoxycyclohexane, polyethylene diglycidyl ether (PEGDE) and 1,2-ethanediol diglycidyl ether.

Embodiment 11: The non-therapeutic use according to Embodiments 9 or 10, wherein the hyaluronic acid composition is provided in the form of gel.

Embodiment 12: The non-therapeutic use according to any one of Embodiments 9-11, wherein the hyaluronic acid composition has a tanδ value of 0.5-1, as determined according to the following Formula 1:

$$[Formula\ 1]$$

$$Tan\delta = G''/G'$$

wherein G" is viscosity of the hyaluronic acid composition, and G' is elasticity of the hyaluronic acid composition.

Embodiment 13: The non-therapeutic use according to any one of Embodiments 9-12, wherein a particle size of the crosslinked hyaluronic acid is 100 μm or more.

Embodiment 14: The non-therapeutic use according to any one of Embodiments 9-13, wherein the hyaluronic acid composition further comprises a solvent in an amount of 20-70 wt% based on the total weight of the hyaluronic acid composition.

Embodiment 15: The non-therapeutic use according to any one of Embodiments 9-14, wherein the hyaluronic acid composition is a cosmetic composition.

**Claims**

1. A hyaluronic acid composition for external use on skin comprising crosslinked hyaluronic acid in an amount of 0.5 wt% or more on dry weight basis, based on the total weight of the hyaluronic acid composition,
   wherein the crosslinked hyaluronic acid has a water swelling degree of 50-100 times, wherein the water swelling degree is a ratio of the weight of gel determined after storing the crosslinked hyaluronic acid in water for 12 hours based on its dry weight.

2. The hyaluronic acid composition for external use on skin according to claim 1, wherein the crosslinked hyaluronic acid is crosslinked with an epoxide-based crosslinking agent,
   preferably,
   the crosslinked hyaluronic acid is crosslinked with at least one selected from the group consisting of 1,4-butanediol diglycidyl ether (BDDE), ethylene glycol diglycidyl ether (EGDGE), 1-(2,3-epoxypropyl)-2,3-epoxycyclohexane, polyethylene diglycidyl ether (PEGDE) and 1,2-ethanediol diglycidyl ether.

3. The hyaluronic acid composition for external use on skin according to claims 1 or 2, wherein the hyaluronic acid composition is provided in the form of gel.

4. The hyaluronic acid composition for external use on skin according to any one of claims 1-3, wherein the hyaluronic acid composition has a tanδ value of 0.5-1, as determined according to the following Formula 1:

$$[\text{Formula 1}]$$

$$\text{Tan}\delta = G''/G'$$

wherein G" is viscosity of the hyaluronic acid composition, and G' is elasticity of the hyaluronic acid composition.

5. The hyaluronic acid composition for external use on skin according to any one of claims 1-4, wherein a particle size of the crosslinked hyaluronic acid is 100 $\mu$m or more.

6. The hyaluronic acid composition for external use on skin according to any one of claims 1-5, wherein the hyaluronic acid composition further comprises a solvent in an amount of 20-70 wt% based on the total weight of the hyaluronic acid composition.

7. The hyaluronic acid composition for external use on skin according to any one of claims 1-6, wherein the hyaluronic acid composition is a cosmetic composition.

8. A non-therapeutic use of a hyaluronic acid composition for skin moisturization,
   wherein the hyaluronic acid composition comprises crosslinked hyaluronic acid in an amount of 0.5 wt% or more on dry weight basis, based on the total weight of the hyaluronic acid composition,
   wherein the crosslinked hyaluronic acid has a water swelling degree of 50-100 times, wherein the water swelling degree is a ratio of the weight of gel determined after storing the crosslinked hyaluronic acid in water for 12 hours based on its dry weight.

9. The non-therapeutic use according to claim 8, wherein the crosslinked hyaluronic acid is crosslinked with an epoxide-based crosslinking agent,
   preferably,
   the crosslinked hyaluronic acid is crosslinked with at least one selected from the group consisting of 1,4-butanediol diglycidyl ether (BDDE), ethylene glycol diglycidyl ether (EGDGE), 1-(2,3-epoxypropyl)-2,3-epoxycyclohexane, polyethylene diglycidyl ether (PEGDE) and 1,2-ethanediol diglycidyl ether.

10. The non-therapeutic use according to claims 8 or 9, wherein the hyaluronic acid composition is provided in the form of gel.

11. The non-therapeutic use according to any one of claims 8-10, wherein the hyaluronic acid composition has a tanδ value of 0.5-1, as determined according to the following Formula 1:

[Formula 1]

$$Tan\delta = G''/G'$$

wherein G" is viscosity of the hyaluronic acid composition, and G' is elasticity of the hyaluronic acid composition.

12. The non-therapeutic use according to any one of claims 8-11, wherein a particle size of the crosslinked hyaluronic acid is 100 $\mu$m or more.

13. The non-therapeutic use according to any one of claims 1-5, wherein the hyaluronic acid composition further comprises a solvent in an amount of 20-70 wt% based on the total weight of the hyaluronic acid composition.

14. The non-therapeutic use according to any one of claims 1-6, wherein the hyaluronic acid composition is a cosmetic composition.

Figure 1

Europäisches
Patentamt
European
Patent Office
Office européen
des brevets

## EUROPEAN SEARCH REPORT

Application Number

EP 20 18 4629

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 102 492 180 A (BEIJING AIMEIKE BIO TECHNOLOGY CO LTD) 13 June 2012 (2012-06-13) | 1-7 | INV. A61K8/73 A61Q19/00 A61Q19/08 A61K31/728 A61K47/36 A61P17/00 A61K8/04 C08L5/08 C08B37/08 |
| Y | * abstract; claims 1-2,5; example 1 * | 8-14 | |
| X | US 2010/136070 A1 (DOBAK JOHN DANIEL [US] ET AL) 3 June 2010 (2010-06-03) | 1-7 | |
| Y | * abstract; claims 1,5,10,13,17 * * paragraphs [0057], [0063], [0070] * | 8-14 | |
| Y | US 2018/193245 A1 (LESHCHINER ADELYA K [US] ET AL) 12 July 2018 (2018-07-12) * abstract; claims 1-2; example 10 * & DATABASE WPI Week 201851 Thomson Scientific, London, GB; AN 2018-59398G & US 2018/193245 A1 (LUROMED LLC) 12 July 2018 (2018-07-12) * abstract * | 8-14 | |
| Y | US 9 693 947 B1 (MARINI JAN L [US] ET AL) 4 July 2017 (2017-07-04) * Summary of the invention; column 3, line 44 - line 56; claims 1,6; example 1 * | 8-14 | TECHNICAL FIELDS SEARCHED (IPC) A61K A61Q A61P C09J C08L C08B |

-/--

The present search report has been drawn up for all claims

| Place of search | Date of completion of the search | Examiner |
|---|---|---|
| Munich | 17 December 2020 | Blott, Catherine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 20 18 4629

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DOLECKOVA I (CONTIPRO BIOTECH S.R.O., DOLNI DOBROUC 401, 561 02 DOLNI DOBROUC, CZECH REPUBLIC, EMAIL: TOMAS.MUTHNY@CONTIPRO.COM) E: "A NEW CROSSLINKED HYALURONIC ACID XEROGEL IN TOPICAL COSMETIC APPLICATIONS", 30 TH IFSCC CONGRESS MUNICH 2018, "COSMETICS: SCIENCE FOR BEAUTY AND LIFESTYLE", 18 -21 SEPTEMBER 2018, INFINITY HOTEL & CONFERENCE RESORT MUNICH, D-85716 UNTERSCHLEISSHEIM, BOOK OF ABSTRACTS, PROCEEDINGS ON DATA CARRIER, THURSDAY 20 SEPTEMBER 2018,, 2018, pages 1-2, XP9524631, * the whole document * | 8-14 | |
| X,P | CN 111 166 686 A (WUXI HUOBAN DAILY-USE CHEMICAL SCIENCE AND TECH CO LTD) 19 May 2020 (2020-05-19) * the whole document * | 1-14 | |
| A | Evonik: "Hyacare Filler CL", , April 2008 (2008-04), XP055444318, Retrieved from the Internet: URL:http://glenncorp.com/wp-content/uploads/2013/11/USA_DS_HyaCare-Filler_10102013.pdf [retrieved on 2018-01-25] * the whole document * | 1-14 | TECHNICAL FIELDS SEARCHED (IPC) |

The present search report has been drawn up for all claims

| Place of search | Date of completion of the search | Examiner |
|---|---|---|
| Munich | 17 December 2020 | Blott, Catherine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**ANNEX TO THE EUROPEAN SEARCH REPORT
ON EUROPEAN PATENT APPLICATION NO.**

EP 20 18 4629

This annex lists the patent family members relating to the patent documents cited in the above-mentioned European search report.
The members are as contained in the European Patent Office EDP file on
The European Patent Office is in no way liable for these particulars which are merely given for the purpose of information.

17-12-2020

| Patent document cited in search report | | Publication date | Patent family member(s) | | Publication date |
|---|---|---|---|---|---|
| CN 102492180 | A | 13-06-2012 | NONE | | |
| US 2010136070 | A1 | 03-06-2010 | US | 2010136070 A1 | 03-06-2010 |
| | | | WO | 2010065784 A2 | 10-06-2010 |
| US 2018193245 | A1 | 12-07-2018 | US | 2010135935 A1 | 03-06-2010 |
| | | | US | 2014286884 A1 | 25-09-2014 |
| | | | US | 2015132237 A1 | 14-05-2015 |
| | | | US | 2015132238 A1 | 14-05-2015 |
| | | | US | 2016213592 A1 | 28-07-2016 |
| | | | US | 2018193245 A1 | 12-07-2018 |
| | | | US | 2020129408 A1 | 30-04-2020 |
| US 9693947 | B1 | 04-07-2017 | NONE | | |
| CN 111166686 | A | 19-05-2020 | NONE | | |

EPO FORM P0459

For more details about this annex : see Official Journal of the European Patent Office, No. 12/82